# EUROPEAN PATENT APPLICATION

(11) **EP 3 042 892 A1**
(43) Date of publication of application: **13.07.2016**
(21) Application number: 15150681.3
(22) Date of filing: 09.01.2015
(51) Int. Cl.: C07C 215/60, C07C 227/34

(54) **Amorphisation of levosalbutamol tartrate**

(71) Applicant: Deva Holding Anonim Sirketi, 34303 Istanbul (TR)
(72) Inventor: Karliga, Bekir, 34303 Istanbul (TR); Bellur Atici, Esen, 34303 Istanbul (TR); Unlu, Zeynep, 34303 Istanbul (TR); Baslar, Yildiray, 34303 Istanbul (TR); Akyil, Suna Ayse, 34303 Istanbul (TR); Sandikci, Tuba, 34303 Istanbul (TR)

(57) **Abstract**

The present invention is directed to method for performing a conversion of a crystalline levosalbutamol tartrate to amorphous levosalbutamol tartrate by spray drying.

The invention further contains aerosol formulation comprising amorphous levosalbutamol tartrate.

## Description

### Technical Field

The present invention is directed to method for performing a conversion of a crystalline levosalbutamol tartrate to amorphous levosalbutamol tartrate by spray drying.

The invention further contains aerosol formulation comprising amorphous levosalbutamol tartrate.

### Background Art

Inhalation is the preferred administration route for respiratory disorders, because the inhaled drug is delivered directly to affected lung tissues. Levosalbutamol is also one of the most preferred medications for treating respiratory disorders administered as aerosol formulations.

Salbutamol was firstly described in US 3644353 B (ALLEN AND HANBURYS LIMITED) 22.02.1972.

Salbutamol has been marketed as a racemic mixture, although the beta 2-agonist activity resides almost exclusively in the (R)-enantiomer.

Levosalbutamol is the active R-isomer of albuterol which contains both the R and S isomers.

It is also known as (R)-Salbutamol or (R)-albuterol, is (R)-α¹-[[(1, 1-dimethylethyl)-amino]methyl-4-hydroxy-1,3 -benzenedimethanol, belongs to the β-2-agonists used pharmaceutically as bronchodilators and is of considerable commercial interest.

The enantioselective disposition of salbutamol and the possibility that (S)-salbutamol has adverse effects have led to the development of enantiomerically pure (R)-salbutamol formulations.

A process for the preparation of optically pure isomer of salbutamol from mono-protected salbutamol precursor is disclosed in US 5545745 (SEPRACOR) 13.08.1996 .

WO 2004/052835 A (SEPRACOR) 24.06.2004 describes a process for preparing levosalbutamol L-tartrate in crystalline form, where Levosalbutamol is prepared by hydrogenating R-benzylsalbutamol in the presence of palladium on carbon. The patent also describes an aerosol formulation adapted for administration using a metered dose inhaler that contains levosalbutamol I-tartrate in crystalline form.

Drugs for treating respiratory and nasal disorders are frequently administered in aerosol formulations through the mouth or nose. An aerosol is defined as a suspension of small particles of liquid or solid in a gas. The pressurized metered dose inhaler (MDI) is the most widely used device for aerosol therapy. The suspension is stored in a sealed container capable of withstanding the pressure required to maintain the propellant as a liquid. The suspension is dispensed by activation of a dose metering valve affixed to the container. Devices used for dispensing drugs in this way are known as "metered dose inhalers" (MDI's). Peter Byron, Respiratory Drug Delivery, CRC Press, Boca Raton, Fla. (1990) for a general background on this form of therapy.

Respiratory disorders are weakening disorders, and they sometimes might be even life threatening, so patients often rely on the drug delivered by metered dose inhalers. Therefore, it is essential that the prescribed dose of aerosol medication delivered to the patient consistently meet the specifications claimed by the manufacturer and comply with the requirements of regulatory authorities. That is, every dose in the canister must be the same within close tolerances.

An aerosol formulation contains at least a pharmaceutical active ingredient and a propellant gas. The propellant gas used in pharmaceutical aerosol formulations is HFA since the use of CFCs was banned due to concerns of ozone destruction. The pharmaceutical aerosol formulations may further contain other excipients such as co-solvent, surfactant and stabilizer.

The medicament in the aerosol formulation is in the form of fine particles. The efficiency of an aerosol inhaler depends on the fine particle dose deposited in the proper site in the lungs. Lungs and airways of the respiratory tract form a particle size-selective system. In this system, larger particles greater than 10 µm cannot reach the lung and are filtered in the oropharynx and are swallowed, particles of 5-10 µm generally reach the proximal generations of the lower respiratory tract and particles smaller than 1 µm are likely to be exhaled. It is generally accepted, that the effective particles should be in the range of 1 to 5 µm in order to reach the distal areas of the respiratory tract.

### Summary of invention

The first object of the present invention is to provide a process to produce levosalbutamol I- tartrate in an amorphous form. The amorphous form of levosalbutamol tartrate is prepared by an efficient process which uses conditions which are convenient to operate on a commercial scale and operationally safe. The provided process comprises the preparation of levosalbutamol tartrate in an amorphous form which comprises dissolving crystalline levosalbutamol tartrate in a suitable solvent or dissolving crystalline levosalbutamol tartrate in a suitable solvent and adding oleic acid to a suitable solvent and recovering amorphous form of levosalbutamol tartrate from the solution thereof by spray drying.

In yet another aspect of this invention there is provided a pharmaceutical composition comprising levosalbutamol tartrate in an amorphous form with one or more pharmaceutical carriers and/or excipients.

### Technical Problem

In aerosol formulations, the particle size distribution is one of the critical parameters in manufacturing of inhalation products, and it directly influences the quality of inhaled drug.

The particle size in aerosol formulations is crucially important for delivering appropriate dose of medicament to patient. If the particle size is under the limit, the patient can not benefit from the inhaler, because the aerosol particles are exhaled. If the particle size is over the limit, excessive medicament can deposit in the oropharyngeal cavity and the drug's absorption can cause undesirable effects either at the systemic level or local level. In aerosol formulation of levosalbutamol, desired particle size is between 1-3 µm to be able to provide the dosage puff adjustment. When amorphous levosalbutamol obtained by spray drying and micronized after spray drying to achieve desired particle size, agglomeration still can occur in the aerosol formulation. Herewith, the process for forming amorphous levosalbutamol is also important.

In pharmaceutical aerosol's manufacturing, homogeneity of aerosol formulation is one of the important parameters. Suspended particles should be homogeneously dispersed in the aerosol formulation in order to deliver a uniform composition. In other words, every dose dispensed from the aerosol canister must be the same within close tolerances.

It is known that agglomeration causes undesired particle size distribution of active ingredient in aerosol formulations. Hence, agglomeration should be avoided in order to obtain a homogeneous composition having particles in desired particle size. Characteristics of API should also be considered during formulation studies; it is known that certain active pharmaceutical ingredient has a strong tendency to agglomerate. The agglomeration and undesired particle size may cause to deliver improper dosages.

Moreover, when any organic solvent other than water used as solvent for spray drying, amorphous levosalbutamol returns into crystalline form in short time in the presence of residual solvent after filled into the canister containing HFA134a as propellant.

The active pharmaceutical ingredient may need to be micronized after spray drying to provide desired particle size, but this process also might cause some other problems. Because of the static electrification and flocculation occurring during micronization process of amorphous levosalbutamol, desired API and formulation properties cannot be achieved and the yield can be low and the process is high costly.

### Solution to Problem

The aim of the present invention is to provide an optimized process to obtain levosalbutamol in amorphous form that overcomes the above-mentioned drawbacks and disadvantages associated with conventional manufacturing methods and utilize it in a stabilized formulation.

Inventors of the present invention have achieved an optimized manufacturing process of amorphous levosalbutamol with an increased stability that stays amorphous after filling into the canister containing a formulation comprising HFA 134a.

The present inventors have now surprisingly developed a new optimized manufacturing process of amorphous levosalbutamol with improved flow properties.

Inventors of the present application managed to develop a new optimized process to obtain levosalbutamol in amorphous form which prevents it from agglomeration in HFA 134a formulation.

Inventors of the present invention investigated effects of addition of oleic acid into the solution to be spray dried and surprisingly developed a process for obtaining amorphous levosalbutamol having better physical properties. They prevented agglomeration and static electrification before and after micronization; provided better particle size distribution and dose uniformity of aerosol formulation.

Crystalline levosalbutamol tartrate is dissolved in oleic acid added water and amorphous levosalbutamol tartrate is formed by spray drying of this solution. According to trials' results, a stable amorphous levosalbutamol tartrate is obtained. When amorphous levosalbutamol tartrate obtained by the method of the present invention is used in aerosol formulations, it is observed that agglomeration is prevented and thus better particle size is achieved. In this way, a proper dose can be delivered to the patient.

Static electrification which occurs during the micronization process performed after spray drying is prevented by the use of oleic acid and it is provided to reach the desired specifications easily. This has offered considerable improvement in yield and provided applicability.

### Description of embodiments

Levosalbutamol is labelled for the treatment or prevention of bronchospasm in patients with reversible obstructive airway disease; it has gained importance in pharmaceutical industry in last years.

Levosalbutamol is marketed under the brand name of Xopenex and is a pressurized metered-dose aerosol inhaler (pMDI), which produces an aerosol for oral inhalation.

A metered dose inhaler comprising an aerosol formulation comprising levosalbutamol L-tartrate in crystalline form is patent protected until 2023 and amorphous levosalbutamol tartrate cannot be provided commercially. Thus there is a need to develop a process to form amorphous levosalbutamol tartrate.

Aspects of the present invention relate to provide an optimized process for manufacturing amorphous levosalbutamol tartrate from crystalline levosalbutamol tartrate suitable for use in pharmaceutical aerosol compositions of the present invention.

It is the first aspect of the present invention to obtain a stable amorphous levosalbutamol tartrate. Correspondingly, a pharmaceutical aerosol formulation of amorphous levosalbutamol tartrate where agglomeration is not observed and proper dose adjustment is made is aimed by the present invention.

In another aspect, the present invention relates to a process for preparing amorphous form of levosalbutamol tartrate by a conventional technique. Such conventional techniques include, but are not limited to, mechanical treatment, heating (quench cooling or desolvation) and various solvent-based processes.

In a more preferred aspect, the present invention relates to a process for preparing amorphous form of levosalbutamol tartrate by a solvent-based process. Solvent-based processes include, but are not limited to, distillation, distillation under reduced pressure or vacuum, rotary evaporation, lyophilisation, precipitation and spray-drying.

In a more preferred aspect, the present invention relates to a process for preparing amorphous form of levosalbutamol tartrate by spray drying technique.

Spray drying is a commonly used method in order to modify the crystal properties, such as particle size, crystal habit, crystallinity content, polymorphism and crystal moisture.

Spray drying is a method for producing a dry powder from a liquid solution or a dispersion of particles in a liquid by drying with a hot gas. An advantage of spray drying is that it enables control of the physical form of the API. The API can be engineered in the spray-drying process to be either crystalline or amorphous depending on the composition of the feedstock and the spray-drying conditions.

Spray drying processes and spray drying equipment are generally described in Perry's Chemical Engineers' Handbook, pages 20-54 to 20-57

(Sixth Edition 1984). More details on spray drying processes and equipment are reviewed by Marshall, "Atomization and Spray-Drying," 50 Chem. Eng. Prog. Monogr. Series 2 (1954), and Masters, Spray drying Handbook (Fourth Edition 1985).

A spray dryer is a device used in spray drying. It takes a liquid stream and separates the solute or suspension as a solid and the solvent into a vapour. The solid is usually collected in a drum or cyclone. The liquid input stream is sprayed through a nozzle into a hot vapour stream and vaporised. Solids forms as moisture quickly leaves the droplets. A nozzle is usually used to make the droplets as small as possible by maximising heat transfer and the rate of water vaporisation. Droplet sizes can range from 20 to 180µm depending on the nozzle.

Spray drying may be performed in a conventional manner in the processes of the present invention. The drying gas used in the invention may be any suitable gas, although inert gases such as nitrogen, nitrogen-enriched air; and argon are preferred. Nitrogen gas is a particularly preferred drying gas for use in the process of the invention.

Preferably, the nitrogen gas of the spray dryer is at an inlet temperature of about 50°C to 200°C. More preferably, the nitrogen gas of the spray dryer is at an inlet temperature of about 100°C to 120°C. The outlet gas temperature used may range from about 35°C to 90°C. Most preferably, the outlet gas temperature used is between 65-85°C.

In an aspect, crystalline levosalbutamol tartrate is dissolved in a suitable solvent and formed into amorphous form by spray drying technique.

In another embodiment of the present invention, suitable solvents include but are not limited to water, water miscible organic solvents and combinations thereof. Suitable water miscible organic solvents include but are not limited to acetone, a C1-C4 alcohol, e.g. methanol, butanol or isopropanol.

Inventors of the present invention surprisingly found that ethanol should not be used in any step of the process; because residual ethanol which is used to dissolve crystalline API and which remains in spray dried amorphous powder causes the reconversion of the amorphous API to crystalline again. In a similar way, other solvents may also influence negatively the amorphous levosalbutamol stability. Thus suitable solvent is selected other than ethanol.

In a more preferred embodiment of this invention, crystalline levosalbutamol tartrate is dissolved in oleic acid added water.

In embodiments of this aspect of the present invention, the oleic acid is used in the range of 1% to 10% weight/weight of the total solution.

In a more preferred embodiment of this aspect of the present invention, the oleic acid is used in amount of 2.5% weight/weight of the total solution.

If necessary, the particles obtained by spray drying can be subjected to micronization or to any other method which is able to reduce their mean size to a value of less than 10µm and preferably of less than 5µm.

Micronization is carried out in devices known as compressed-air micronizer or fluid jet mill. In these devices, the particles are carried by a strong stream of air into a chamber designed so that the particles are subjected therein to a large number of impacts.

In another aspect, this invention provides a spray drying process for converting crystalline levosalbutamol tartrate to a corresponding essentially amorphous levosalbutamol tartrate with proviso that ethanol is not used.

In another aspect, this invention provides aprocessfor the preparation of amorphous levosalbutamol tartrate comprises; (a) dissolving crystalline levosalbutamol tartrate in a suitable solvent; (b) adding one or more additional solvents before removing the solvent in step (a); (c) recovering crystalline levosalbutamol tartrate from said solution by spray drying.

The following examples are given as the purpose of illustration of the present invention and should not limit the scope of the invention.

### Example: Active pharmaceutical Ingredient's Related Examples:

### Example-1: Obtaining amorphous levosalbutamol tartrate from crystalline levosalbutamol tartrate dissolved in water.

Crystalline levosalbutamol tartrate (10 g) is dissolved in 30 ml of water at ambient temperature in a beaker by mixing on a magnetic stirrer continuously. Approximately 7-9 g amorphous levosalbutamol tartrate is obtained after spray drying of the solution.

Spray drying parameters are as follows: inlet temperature: 100-120°C, outlet temperature: 65-85 °C, airflow 28 m³/h and solution feed rate of approximately 0.8 l/h.

Particle size of obtained powder is decreased to a value of d (0.9) <3 µm by micronizer. The results are given in Table-1.

### Example-2: Obtaining amorphous levosalbutamol tartrate from crystalline levosalbutamol tartrate by dissolving in oleic acid added water.

Crystalline levosalbutamol (10 g) is dissolved in 30 ml of water in a beaker at ambient temperature by mixing on a magnetic stirrer at high speed continuously. 0.25 g oleic acid was added to the solution and the solution was stirred on a magnetic stirrer at high speed. Approximately 7-9 g amorphous levosalbutamol tartrate is obtained after spray drying.

Spray drying parameters are as follows: gas inlet temperature: 100-120°C, outlet temperature: 65-85 °C, airflow 28 m³/h and solution feed rate of approximately 0.8 l/h.

Particle size of obtained powder is decreased to a value of d (0.9) <3 µm by micronizer. The results are given in Table-1.

### Example-3: Obtaining amorphous levosalbutamol tartrate from crystalline levosalbutamol tartrate dissolved in water and ethanol.

Crystalline levosalbutamol tartrate (10 g) is dissolved in 20 ml of water and 20 ml of ethanol in a beaker at ambient temperature by mixing on a magnetic stirrer continuously. Approximately 7-9 g amorphous levosalbutamol tartrate is obtained after spray drying.

Spray drying parameters are as follows: gas inlet temperature: 100-120°C, gas outlet temperature: 65-85 °C, airflow 28 m³/h and solution feed rate of approximately 0.8 l/h.

Particle size of obtained powder is decreased to a value of d (0.9) <3 µm by micronizer. The results are given in Table-1.

### Example-4: Obtaining amorphous levosalbutamol tartrate from crystalline levosalbutamol tartrate dissolved in oleic acid added water and ethanol.

Crystalline levosalbutamol tartrate (10 g) is dissolved in 20 ml of water and 20 ml of ethanol in a beaker at ambient temperature by mixing on a magnetic stirrer at high speed continuously. 0.25 g oleic acid is added to the solution and the solution is stirred on a magnetic stirrer at high speed continuously. Approximately 7-9 g of amorphous levosalbutamol tartrate is obtained after spray drying.

Spray drying parameters are as follows: gas inlet temperature: 100-120°C, gas outlet temperature: 65-85 °C, airflow 28 m³/h and solution feed rate of approximately 0.8 l/h.

Particle size of obtained powder is decreased to a value of d (0.9) <3 µm by micronizer. The results are given in Table-1.

**Table 1**

| Sample Name / Specification | Physical Situation Results | | | Micronization Results | | |
|---|---|---|---|---|---|---|
| | Stability | Hygrosc opicity | Agglomeration | Static Electrification | Achieving Desired PSD | Result |
| Ex -1 | unstable | yes | yes | yes | Hard | Not Suitable |
| Ex -2 | stable | no | no | no | easy | Suitable |
| Ex -3 | unstable | yes | yes | yes | hard | Not Suitable |
| Ex -4 | unstable | yes | yes | yes | hard | Not Suitable |

Stability study for the active pharmaceutical ingredients obtained in all examples is carried out by storing the APIs at ambient temperature and humidity. According to short-term and long-term stability tests only Example 2 is found stable.

The hygroscopicity of all examples is measured by Karl Fischer titration and found that all APIs except for Example 2 take moisture, which is undesired.

The particle size distribution (PSD) is determined for each API and desired particle size distribution is achieved easily with Example 2.

The static electrification and agglomeration are evaluated by making visual test periodically. In Example 2, no agglomeration and static electrification of API is observed.

When results of Example-1 and Example-2 are compared, it is seen that use of oleic acid in solvent for spray drying, has increased the stability and improved the physical properties.

When the results of Example-3 and Example-4 are evaluated, it is observed that the results of the experiment at which only water and oleic acid is used, could not be reached.

In another aspect of the present invention, the amorphous levosalbutamol tartrate dry powders produced by spray drying may be formulated as active drug in any drug formulations.

In an embodiment of this aspect, amorphous levosalbutamol tartrate may be administered with either a DPI, in suspension or solution with a suitable propellant with a pMDI.

In a preferred embodiment of this aspect, amorphous levosalbutamol tartrate may be administered in a suspension with a suitable propellant with a pMDI.

The aerosol compositions of the present invention may include co-solvents and/or stabilizers and/or surfactants. In suspension formulations, it is also a major drawback that the suspended particles accumulate over time (e.g. on storage) and become unstable. Other inactive ingredients may be added in appropriate amounts for keeping the formulation stable. Stabilizers and co-solvents are investigated in order to overcome the stability problem in many researches. However, sometimes it is more desirable not to use formulation excipients like surfactants, co-solvents etc in order to obtain a taste and odour free, less irritant and less toxic formulations.

A co-solvent may be included to aid to help dissolve or solubilise the pharmaceutical active ingredient in the propellant. It also improves miscibility of surfactants in the propellant and reduces the vapour pressure of the propellant system. Suitable co-solvents include, but are not limited to, water, ethers, alcohols, hydrocarbons. More specifically, co-solvent may comprise acetone, isopropanol, propylene glycol, dipropylene glycol, ethyl acetate, hexylene glycol, and acetone. Although ethanol is the most preferred co-solvent in pharmaceutical aerosol formulations, inventors of the present invention surprisingly found that ethanol should not be used in aerosol formulations of the present invention, due to the fact that it causes the reconversion of amorphous API to become crystalline again. In a similar way, other co-solvents may also influence the amorphous levosalbutamol stability negatively.

A surfactant is used to help improve the solubility of the active ingredient or homogeneity of the dispersion. Surfactants also may help to aid in valve lubrication. Surfactants include, but are not limited to, sorbitan trioleate, lecithin, oleic acid, ethyl oleate, citric acid, benzalkonium chloride, isopropyl myristate, polyvinylpyrrolidone, polyoxypropylene/polyoxyethylene block copolymers, ethoxylated castor oil and the like. The most preferred and typical surfactants allowed by the FDA include sorbitan trioleate, lecithin and oleic acid.

A stabilizer may be added to stabilize the formulation in order to prevent the creaming, flocculating or settling of the formulation. Stabilizers include, but are not limited to, amino acid, mineral acid cyclodextrin and water.

In a most preferred embodiment, crystalline levosalbutamol tartrate is dissolved in oleic acid added water and then spray dried .The most preferred aerosol formulation according to invention contains only HFA 134a and API.

The following examples are given as the purpose of illustration of the present invention and should not limit the scope of the invention.

### Example : Formulation Related Examples:

### Example-5: The formulation containing ethanol, HFA 134a and API obtained in Example-1

By using related amorphous API, the filling into canisters is done with absolute ethanol used as co-solvent and HFA 134a. Later, dose uniformity of aerosol formulation was measured and XRD analysis about polymorphic structure was done with the sample discharged from the canister. The results are given in Table-2.

### Example-6: The formulation containing HFA 134a and API obtained in Example-1

By using related amorphous API, the filling into canisters is done with HFA 134a. Later, dose uniformity of aerosol formulation is measured and XRD analysis about polymorphic structure is done with the sample discharged from the canister. The results are given in Table-2.

### Example-7 The formulation containing ethanol, HFA 134a and API obtained in Example-2

By using related amorphous API, the filling into canisters is done with absolute ethanol used as co-solvent and HFA 134a. Later, dose uniformity of aerosol formulation is measured and XRD analysis about polymorphic structure is done with the sample discharged from the canister. The results are given in Table-2.

### Example-8: The formulation containing HFA 134a and API obtained in Example-2

By using related amorphous API, the filling into canisters is done with HFA 134a. Later, dose uniformity of aerosol formulation is measured and XRD analysis about polymorphic structure is done with the sample discharged from the canister. The results are given in Table-2.

### Example-9: The formulation containing ethanol, HFA 134a and API obtained from Example-3

By using related amorphous API, the filling into canisters is done with absolute ethanol used as co-solvent and HFA 134a. Later, dose uniformity of aerosol formulation is measured and XRD analysis about polymorphic structure is done with the sample discharged from the canister. The results are given in Table-2.

### Example-10: The formulation containing HFA 134a and API obtained from Example-3

By using related amorphous API, the filling into canisters is done with HFA 134a. Later, dose uniformity of aerosol formulation is measured and XRD analysis about polymorphic structure is done with the sample discharged from the canister. The results are given in Table-2.

Spray drying parameters are as follows: inlet temperature: 100-120°C, outlet temperature: 65-85 °C, airflow 28 m³/h and solution feed rate of approximately 0.8 l/h.

Particle size of obtained powder is decreased to a value of d (0.9) <3 µm by micronizer.

By using related amorphous API, the filling into canisters is done with HFA134a. Then, the dose uniformity of aerosol formulation is measured and XRD analysis about polymorphic structure is done with the sample discharged from the canister.

The results are given in Table-2.

**Table 2**

| API used in Formulation | Composition of the formulation-Absolute Ethanol | Stability | PSD | Dose Uniformity | Poly morphism | Result |
|---|---|---|---|---|---|---|
| API obtained in Ex-1 | used | Not stable. Highly hygroscopic. Adhesion and agglomeration was observed | Not stable Increasing by agglomeration | Cannot be achieved depending on PSD | Turns into the crystalline form due to the use of ethanol | Not suitable |
| API obtained in Ex-1 | not used | Not stable. Highly hygroscopic. Adhesion and agglomeration was observed | Not stable Increasing by agglomeration | Cannot be achieved depending on PSD | Stays amorphous | Not suitable |
| API obtained in Ex-2 | used | Stable. No adhesion and agglomeration was observed. Fluidity is good | Not stable Increases while turning into crystalline form | Cannot be achieved depending on PSD | Turns into the crystalline form due to the use of ethanol | Not suitable |
| API obtained in Ex-2 | not used | Stable | Stabled (0,9) <3 micrn | Achieved | Stays amorphous | Suitable |
| API obtained in Ex-3 | used | Not stable. Highly hygroscopic. Adhesion and agglomeration was observed | Not stabl e Incre ases while turning into crystalline form | Cannot be achieved depending on PSD | Turns into the crystalline form due to the use of ethanol | Not suitable |
| API obtained in Ex-3 | not used | Not stable. Highly hygroscopic. Adhesion and agglomeration was observed | Not stable Increases while turning into crystalline form | Cannot be achieved depending on PSD | Turns into the crystalline form with residual ethanol | Not suitable |

Stability study for the formulations is carried out by storing the formulations at ambient temperature and humidity. According to short-term and long-term stability tests, Example 8 is found stable, when others not stable.

The particle size distribution is determined using Malvern "Mastersizer 3000"The particle size distribution (PSD) is determined for each formulation and Example 8 gave best results. In other formulations, agglomeration is observed and PSD increased with the effect of agglomeration.

The dose uniformity measurements for all formulation examples are made and observed that desired dose uniformity is achieved in Example 8.

The atomic structure is studied by XRD and observed that API in Example 8 stays amorphous, when API in other formulations turns into crystalline form with the effect of ethanol used in formulation or spray drying solution.

As a result, it is seen that the inhaler product is stable and has a proper dose amount when formulated without ethanol but only HFA 134 a (1, 1, 1, 2-tetrafluoroethane) and the API gave best results which was dissolved in 2.5% (by weight) oleic acid added water before spray drying.

When all results of API and formulation examples evaluated, the inventors of the present invention found oleic acid addition into the solution to be spray dried improved both API and formulation properties and they also found that ethanol should not used in any step of the process. Ethanol used for dissolving the crystalline levosalbutamol tartrate before spray drying may remain in dry powder and causes amorphous levosalbutamol to form crystalline again. Likewise, ethanol used as co-solvent in aerosol formulation causes amorphous levosalbutamol to form crystalline again.

## Claims

1. A spray drying process for converting crystalline levosalbutamol tartrate to a corresponding essentially amorphous levosalbutamol tartrate with proviso that ethanol is not used.

2. The process, according to claim 1, for the preparation of amorphous levosalbutamol tartrate comprises; (a) dissolving crystalline levosalbutamol tartrate in a suitable solvent; (b) adding one or more additional solvents before removing the solvent in step (a); (c) recovering crystalline levosalbutamol tartrate from said solution by spray drying.

3. The suitable solvent, according to claim 2, is water,

4. The additional solvent added to water, according to claim 2, is oleic acid.

5. In the spray drying process, according to claim 2, the gas inlet temperature ranges from 40°C to 100°C.

6. The process according to claim 2, wherein the gas outlet temperature of the spray drier ranges from 20°C to 80°C.

7. Obtained amorphous levosalbutamol tartrate, acccording to preceding claims, is used in an inhalation formulations with proviso that ethanol is not used in said formulation.
